Europäisches Patentamt

European Patent Office

Office européen des brevets

(19)

(11) Veröffentlichungsnummer : **0 074 596**
**B1**

(12) **EUROPÄISCHE PATENTSCHRIFT**

(45) Veröffentlichungstag der Patentschrift :
28.08.85

(21) Anmeldenummer : 82108199.9

(22) Anmeldetag : 06.09.82

(51) Int. Cl.⁴ : **G 21 K   1/02,** H 05 G   1/02,
A 61 B   6/06

(54) **Röntgenuntersuchungsgerät.**

(30) Priorität : **16.09.81 DE 3136806**

(43) Veröffentlichungstag der Anmeldung :
**23.03.83 Patentblatt 83/12**

(45) Bekanntmachung des Hinweises auf die Patenterteilung : **28.08.85 Patentblatt 85/35**

(84) Benannte Vertragsstaaten :
**BE DE FR IT**

(56) Entgegenhaltungen :
**DE-A- 2 728 998**
**DE-A- 2 744 808**
**DE-A- 2 932 042**
**FR-A- 2 440 014**
**US-A- 2 942 126**
**US-A- 4 097 748**

(73) Patentinhaber : **Siemens Aktiengesellschaft**
**Berlin und München Wittelsbacherplatz 2**
**D-8000 München 2 (DE)**

(72) Erfinder : **Goldmann, Norbert**
**Mühlweg 21 A**
**D-8521 Uttenreuth (DE)**

**Beschreibung**

Die Erfindung bezieht sich auf ein Röntgenuntersuchungsgerät mit einer Röntgenröhre mit mindestens zwei verschiedenen, wahlweise einschaltbaren Brennflecken, mit einer zur Röntgenröhre justierten Primärstrahlenblende und mit einer Bildschicht.

Es ist in der Röntgentechnik üblich, Drehanodenröhren so auszubilden, daß der Anodenteller zwei gegenüber der Tellerebene verschieden stark geneigte Brennfleckbahnen besitzt. Jeder dieser Brennfleckbahnen ist dann eine eigene Kathode zugeordnet. Weil der auf der gegenüber der Tellerebene schächer geneigten Brennfleckbahn erzeugte Brennfleck in der radialen Projektionsrichtung, d. h. von der Bildschicht gesehen kleinere axiale Abmessungen zeigt, wird er bei gleichzeitiger Verringerung seiner Abmessungen in der dazu senkrechten Richtung, was durch entsprechende Dimensionierung der Kathode erreicht wird, vorzugsweise zur Erzeugung feinzeichnender Röntgenaufnahmen verwendet. Wegen seiner kleineren Fläche ist jedoch seine maximal zulässige Dosisleistung geringer als jene des anderen Brennflecks. Mit der der stärker geneigten Brennfleckbahn zugeordneten Kathode wird im allgemeinen auch ein größerer Brennfleck erzeugt, so daß man auch mit einer höheren Dosisleistung arbeiten kann. Es ist eine Eigenart solcher Röntgenuntersuchungsgeräte, daß beide Brennflecke an verschiedenen geometrischen Orten entstehen und das Umschalten von dem einen zum anderen Brennfleck zu einer Dejustierung der Primärstrahlenblende führt. Ähnlich wie bei einer Parallaxenverschiebung hat das eine zwar geringe, aber doch merkliche Verschiebung des Bildbereichs auf der Bildschichtebene zur Folge. Diese Bildverschiebung wird naturgemäß um so größer, je weiter die beiden Brennflecke auseinanderliegen und je kleiner der Zahlenwert des Bruches ist, der sich aus dem Verhältnis der Abstände Brennfleck-Blende zum Abstand Blende-Bildschicht ergibt.

Der Erfindung liegt die Aufgabe zugrunde, einen Weg zu weisen, wie die Bildverschiebung in der Bildschichtebene beim Wechsel des Brennflecks mit geringstmöglichem Aufwand verringert werden kann.

Bei einem Röntgenuntersuchungsgerät der eingangs genannten Art werden daher erfindungsgemäß die Blendenplatten der Primärstrahlenblende beim Umschalten von dem einen zum anderen Brennflecksynchron in der gleichen Richtung und um etwa den gleichen Betrag verschoben, um den sich der Brennfleck verschiebt. Dies hat zur Folge, daß die Bildverschiebung in der Bildschichtebene nicht größer wird als die Verschiebung des Brennflecks selbst.

Eine weitere Verringerung der Bildverschiebung in der Bildschichtebene beim Umschalten von dem einen auf den anderen Brennfleck läßt sich erreichen, wenn die Verschiebung in Weiterbildung der Erfindung in etwa um das Verhältnis des Abstandes der Brennflecke von den Blendenplatten der Primärstrahlenblende zum Abstand dieser Blendenplatten von der Bildschicht kleiner gehalten ist als die durch die Brennfleckumschaltung bewirkte Brennfleckverschiebung. Durch eine solche Verringerung des Stellwegs der Blendenplatten kann die Bildverschiebung in der Bildschichtebene beim Umschalten von dem einen zum anderen Brennfleck sogar ganz verhindert werden.

Eine einfache nachrüstbare Konstruktion ergibt sich, wenn die gesamte Primärstrahlenblende in Ausgestaltung der Erfindung gegenüber ihrer Halterung verschoben wird. Bei dieser Konstruktion lassen sich auch handelsübliche Primärstrahlenblenden verwenden.

In zweckmäßiger Ausgestaltung der Erfindung kann der Blendeplattenträger im Gehäuse der Primärstrahlenblende verschoben werden. Diese Konstruktion ist nicht nur optisch ansprechender als die vorgenannte Ausführungsform, sie ist auch weniger störanfällig, weil die Stellelemente im Blendengehäuse geschützt untergebracht sind.

Weitere Einzelheiten der Erfindung werden anhand zweier in den Figuren dargestellter Ausführungsbeispiele erläutert. Es zeigen:

Figur 1 eine schematische Darstellung des Strahlenverlaufs bei einem erfindungsgemäßen Röntgenuntersuchungsgerät, bei dem die Primärstrahlenblende insgesamt verschiebbar ist,

Figur 2 einen Schnitt durch eine Primärstrahlenblende, bei der die Symmetrieachse der Einblendung innerhalb des Blendengehäuses verschiebbar ist, und

Figur 3 einen Schnitt längs der Linie III-III der Figur 2.

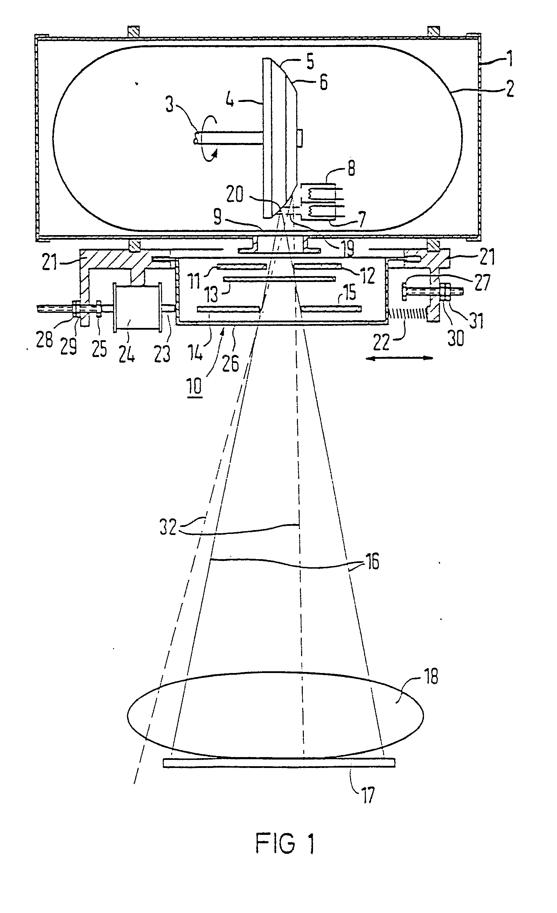

In der Fig. 1 erkennt man eine in einem metallenen Röhrengehäuse 1 gelagerte Röntgenröhre 2. Im Innern der Röntgenröhre 2 ist ein um eine Achse 3 drehbarer Anodenteller 4 mit zwei unterschiedlich zur Tellerebene geneigten Brennfleckbahnen 5, 6 und zwei jeweils einer Brennfleckbahn zugeordneten Kathoden 7, 8 eingezeichnet. Vor dem Strahlenaustrittsfenster 9 des Röhrengehäuses 1 ist eine Primärstrahlenblende 10 parallel zur Achse 3 des Anodentellers 4 verschiebbar am Röhrengehäuse 1 gelagert. Im Inneren der Primärstrahlenblende 10 sind die in verschiedenen Ebenen verschiebbaren Blendenplatten 11, 12, 13, 14, 15 angedeutet. In dem durch die Blendenplatten ausgeblendeten Strahlenkegel 16 ist eine Bildschicht 17 angeordnet. Die Bildschicht steht hier wahlweise für einen Leuchtschirm eines Röntgenbildverstärkers oder ein in einer Röntgenfilmkassette eingelegtes Filmblatt. Zwischen der Bildschicht 17 und der Primärstrahlenblende 10 ist der zu untersuchende Patient 18 angedeutet. In der Darstellung der Fig. 1 ist die der äußeren Brennfleckbahn zugeordnete Kathode 7 eingeschaltet dargestellt. Der Elektronenstrahl 19 ist gepunktet dargestellt. Der vom Brennfleck 20 ausgehende, durch die Blendenplatten auf die Bildschicht 17 eingeblendete Strahlenkegel 16 ist durch zwei durchgehende Linien angedeutet.

Die Primärstrahlenblende ist, wie die Fig. 1 erkennen läßt, in einer am Röhrengehäuse 1 befestigten rechteckigen flanschartigen Halterung 21 parallel zur Achse 3 des Anodentellers 4 verschiebbar gelagert. Sie wird von einer Druckfeder 22 in eine Extremstellung gedrückt, in der sie den Anker 23 eines Hubmagneten 24 gegen einen Anschlag 25 drückt. Bei Erregung des Hubmagneten 24 kann der Anker 23 das Gehäuse 26 der Primärstrahlenblende 10 entgegen der Kraft der Druckfeder 22 gegen einen weiteren Anschlag 27 zur Anlage bringen. Beide Anschläge 25, 27 sind über Kontermuttern 28, 29, 30, 31 einstellbar.

Beim Durchleuchtungsbetrieb, wenn die der stärker geneigten Brennfleckbahn 5 zugeordnete Kathode 7, wie in Fig. 1 dargestellt, eingeschaltet ist, bleibt der Hubmagnet 24 stromlos. Das Gehäuse 26 der Primärstrahlenblende 10 wird durch die Druckfeder 22 in der Darstellung der Fig. 1 nach links in eine Extremstellung gedrückt gehalten, bei der es den Anker 23 des Hubmagneten 24 gegen den linken Anschlag 25 drückt. Der vom Brennfleck ausgehende Röntgenstrahlenkegel 16 ist über diesen Anschlag 25 so zur Bildschicht 17 einstellbar, daß sich bei entsprechend weiter Öffnung der Blendenplatten 11, 12, 13, 14, 15 das gesamte Feld der Bildschicht 17 ohne Randüberschneidungen exakt ausleuchten läßt.

Soll nun der Untersuchungsbefund in einer Röntgenaufnahme festgehalten werden, so kann zur Untersuchung feinerer Details auf die der schwächer geneigten Brennfleckbahn 6 zugeordneten Kathode 8 umgeschaltet werden. Der so entstehende Strahlenkegel 32 würde bei unverschobener Primärstrahlenblende 10 die in der Figur gestrichelt angedeutete Kontur haben und ein anderes Strahlenfeld auf der Bildschicht ausleuchten. Um dies zu vermeiden, wird synchron mit der anderen Kathode 8 auch der Hubmagnet 24 mit an Spannung gelegt. Dieser drückt mit seinem Anker 23 das Gehäuse 26 der Primärstrahlenblende 10 entgegen der Kraft der Druckfeder 22 gegen den in der Fig. 1 rechten einstellbaren Anschlag 27. Dieser Anschlag ist so justiert, daß der durch den Wechsel des Brennflecks entstehende neue Strahlenkegel weniger stark verschoben ist als bei nicht verschobener Primärstrahlenblende 10. Bei der Verschiebung der Primärstrahlenblende 10 um einen bestimmten Betrag nach rechts, in der Darstellung der Fig. 1, kann sogar erreicht werden, daß der neue von dem Auftreffpunkt der Elektronen auf der schwächer geneigten Brennfleckbahn 6 ausgehende Strahlenkegel in der Bildschichtebene in der gleichen Bildumgrenzung auftrifft wie der ausgezogene Strahlenkegel 16.

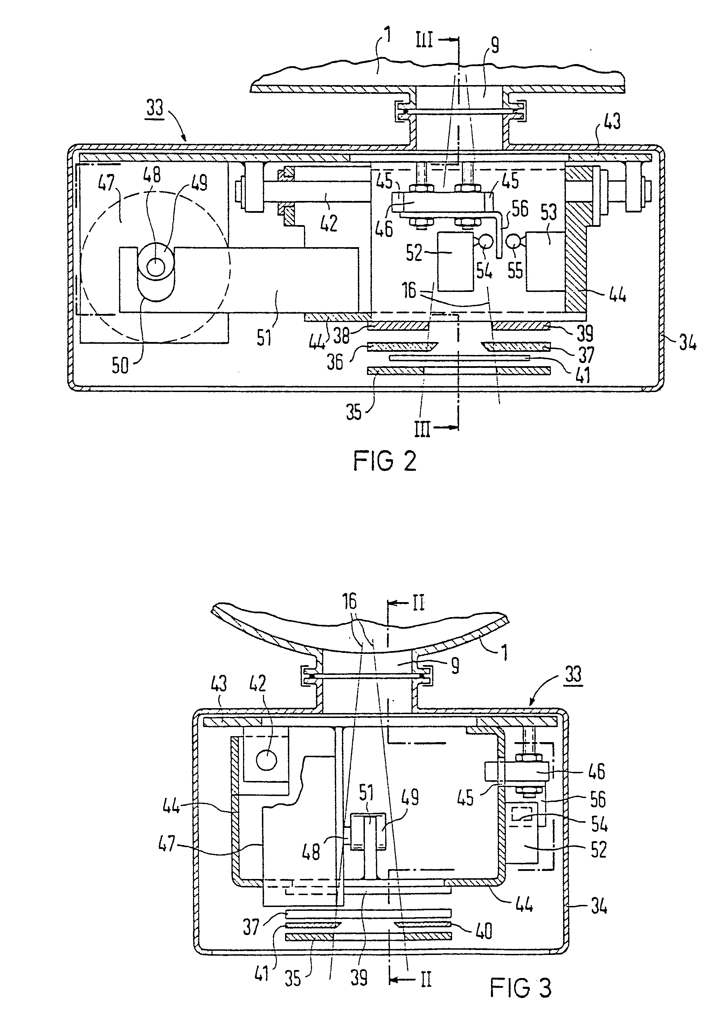

Die Fig. 2 und 3 zeigen eine andere Primärstrahlenblende 33, bei der das Blendengehäuse 34 unverrückbar fest am Röntgenröhrengehäuse 1 angeflanscht bleiben kann und dafür die Blendeplatten 35, 36, 37, 38, 39, 40, 41 in ihrer Gesamtheit im Blendengehäuse 34 der Primärstrahlenblende 33 verschiebbar sind. In der Fig. 2 erkennt man, daß eine Führungsstange 42 auf einer Grundplatte 43 der Primärstrahlenblende 33 montiert ist, an der ein tunnelartiger Blendenträger 44 längsverschiebbar gelagert ist. Auf der der Führungsstange 42 gegenüberliegenden Seite trägt der Blendenträger 44, wie die Fig. 3 zeigt, einen parallel zur Führungsstange ausgerichteten Längsschlitz 45. In diesen Längsschlitz ragt ein an der Grundplatte 43 höhenverstellbar gelagertes Kunststoffgleitstück 46 hinein. Neben dem Blendenträger 44 ist ein Getriebemotor 47 auf der Grundplatte 43 befestigt. Auf dessen Abtriebswelle 48 ist ein Exzenter 49 montiert. Der Exzenter ragt in ein Langloch 50 einer Mitnehmerplatte 51 hinein. Diese ist mit dem Blendenträger 44 verbunden. Das Langloch 50 ist so breit, daß der Exzenter 49 nahezu spielfrei darin drehbar ist. Auf dem Blendenträger 44 sind in hier nicht weiter dargestellter Weise die Stelltriebe für die einzelnen Blendenplatten 35, 36, 37, 38, 39, 40, 41 montiert. Außerdem sind am Blendenträger zwei Mikroschalter 52, 53 befestigt, deren Schaltrollen 54, 55 gegen einen auf der Grundplatte 43 montierten Schaltwinkel 56 arbeiten.

Die in den Fig. 2 und 3 dargestellte Primärstrahlenblende 33 kann unverrückbar am flanschartig ausgebildeten Strahlenaustrittsfenster 9 des Röhrengehäuses 1 befestigt werden. Beim Umschalten des Betriebs der Röntgenröhre 2 von dem einen Brennfleck zum anderen wird der Getriebemotor 47 über den jeweils anderen Mikroschalter 52, 53 eingeschaltet. Der Getriebemotor dreht nun den Exzenter 49 und verschiebt mit dem Exzenter die Mitnehmerplatte 51 und den Blendenträger 44 so lange, bis der an Spannung liegende Mikroschalter ausschaltet und den Getriebemotor 47 stillsetzt. Die auf den Blendenträger montierten Blendenplatten 35, 36, 37, 38, 39, 40, 41 mitsamt ihren Stellmechanismen sind auf diese Weise so zum gewählten neuen Brennfleck 20, 31 zentriert, daß der ausgeblendete Strahlenkegel 16 in der Bildschichtebene auf die gleichen Flächenelemente fällt, wie vor dem Brennfleckwechsel. Die Einstellung der Blendenplatten auf ein bestimmten Film- oder Bildformat bleibt hiervon unberührt. Durch Verschieben der Mikroschalter 52, 53 relativ zum Blendenträger 44 lassen sich die Haltepunkte des Getriebemotors 47 auch so legen, daß der gegenseitige Abstand der Halteposition kleiner ist als der maximale Hub des Exzenters.

**Patentansprüche**

1. Röntgenuntersuchungsgerät mit einer Röntgenröhre mit mindestens zwei verschiedenen, wahlweise einschaltbaren Brennflecken, mit einer zur Röntgenröhre justierten Primärstrahlenblende und mit einer Bildschicht, dadurch gekenzeichnet, daß die Blendenplatten (11, 12, 13, 14, 15, 35, 36, 37, 38, 39, 40, 41) der Primärstrahlenblende (10, 33) beim Umschalten von dem einen zum anderen Brennfleck (20) synchron in der gleichen Richtung und um etwa den gleichen Betrag verschoben werden, um den sich der Brennfleck verschiebt.

2. Röntgenuntersuchungsgerät nach Anspruch 1, dadurch gekennzeichnet, daß die Verschiebung in etwa um das Verhältnis des Abstandes der Brennflecke (20) von den Blendenplatten (11 bis 15, 35 bis 41) der Primärstrahlenblende (10, 33) zum Abstand dieser Blendenplatten von der Bildschicht (17) kleiner gehalten ist als die durch die Brennfleckumschaltung bewirkte Brennfleckverschiebung.

3. Röntgenuntersuchungsgerät nach Anspruch 1, dadurch gekennzeichnet, daß die gesamte Primärstrahlenblende (10) gegenüber ihrer Halterung (21) verschoben wird.

4. Röntgenuntersuchungsgerät nach Anspruch 1, dadurch gekennzeichnet, daß der Blendenplattenträger (44) im Gehäuse (34) der Primärstrahlenblende (33) verschoben wird.

5. Röntgenuntersuchungsgerät nach Anspruch 3 oder 4, dadurch gekennzeichnet, daß der Verschiebeweg durch Anschläge (25, 27) begrenzt ist.

6. Röntgenuntersuchungsgerät nach Anspruch 3 oder 4, dadurch gekennzeichnet, daß die Verschiebung über einen Hubmagneten (24) erfolgt.

7. Röntgenuntersuchungsgerät nach Anspruch 3 oder 4, dadurch gekennzeichnet, daß die Verschiebung über einen Exzenter (49) erfolgt.

8. Röntgenuntersuchungsgerät nach Anspruch 7, dadurch gekennzeichnet, daß der Exzenter (49) elektromotorisch gedreht wird.

## Claims

1. X-ray examination apparatus comprising an X-ray tube having at least two different focal spots which can be selectively switched on, a primary beam diaphragm which is adjusted relative to the X-ray tube, and an image layer, characterised in that when switch-over takes place from one focal spot to the other (20), the diaphragm plates (11, 12, 13, 14, 15, 35, 36, 37, 38, 39, 40, 41) of the primary beam diaphragm (10, 33) are displaced in synchronism in the same direction and by approximately the same amount as that by which the focal spot is displaced.

2. X-ray examination apparatus as claimed in Claim 1, characterised in that the displacement by approximately the ratio of the distance of the focal spot (20) from the diaphragm plates (11 to 15, 35 to 41) of the primary beam diaphragm (10, 33) to the distance of these diaphragm plates from the image layer (17) is kept smaller than the focal spot displacement produced by switch-over of the focal spot.

3. X-ray examination apparatus as claimed in Claim 1, characterised in that the entire primary beam diaphragm (10) is displaced relative to its support (21).

4. X-ray examination apparatus as claimed in Claim 1, characterised in that the diaphragm plate carrier (44) is displaced in the housing (34) of the primary beam diaphragm (33).

5. X-ray examination apparatus as claimed in Claim 3 or Claim 4, characterised in that the displacement travel is limited by stop means (25, 27).

6. X-ray examination apparatus as claimed in Claim 3 or Claim 4, characterised in that the displacement is effected by means of a travelling magnet (24).

7. X-ray examination apparatus as claimed in Claim 3 or Claim 4, characterised in that the displacement is effected by means of an eccentric (49).

8. X-ray examination device as claimed in Claim 7, characterised in that the eccentric (49) is driven by an electric motor.

## Revendications

1. Appareil de radiodiagnostic comportant un tube à rayons X muni d'au moins deux foyers différents pouvant être branchés à volonté, et un diaphragme du rayonnement primaire réglé par rapport au tube à rayons X et une couche d'image, caractérisé par le fait que les plaques collimatrices (11, 12, 13, 14, 15, 35, 36, 37, 38, 39, 40, 41) du diaphragme du rayonnement primaire (10, 33) sont déplacées en synchronisme, lors du passage d'un foyer à l'autre foyer (20), suivant la même direction et sur approximativement la même distance que celle sur laquelle se déplace le foyer.

2. Appareil de radiodiagnostic suivant la renvendication 1, caractérisé par le fait que le déplacement est conservé inférieur au déplacement du foyer, provoqué par le changement de foyer, et ce approximativement dans le rapport de la distance du foyer (20) aux plaques collimatrices (11, 15, 35 à 41) du diaphragme du rayonnement primaire (10, 33) à la distance de ces plaques de diaphragme par rapport à la couche d'image (17).

3. Appareil de radiodiagnostic suivant la revendication 1, caractérisé par le fait que l'ensemble du diaphragme du rayonnement primaire (10) est décalé par rapport à son support (21).

4. Appareil de radiodiagnostic suivant la revendication 1, caractérisé par le fait que le support (44) des plaques collimatrices est décalé dans le boîtier (34) du diaphragme du rayonnement primaire (33).

5. Appareil de radiodiagnostic suivant la revendication 3 ou 4, caractérisé par le fait que le trajet de déplacement est limité par les butées (25, 27).

6. Appareil de radiodiagnostic suivant la revendication 3 ou 4, caractérisé par le fait que le déplacement est réalisé par l'intermédiaire d'un aimant de levage (34).

7. Appareil de radiodiagnostic suivant la revendication 3 ou 4, caractérisé par le fait que le déplacement est réalisé au moyen d'un excentrique (49).

8. Appareil de radiodiagnostic suivant la revendication 7, caractérisé par le fait que l'excentrique (49) est entraîné en rotation par un moteur électrique.

FIG 1

FIG 2

FIG 3